# EUROPÄISCHE PATENTSCHRIFT

(11) **EP 0 529 122 B1**
(45) Veröffentlichungstag und Bekanntmachung des Hinweises auf die Patenterteilung: **08.11.1995**
(21) Anmeldenummer: 91114252.9
(22) Anmeldetag: 26.08.1991
(51) Int. Cl.: A61N 1/365, A61N 1/08, G01R 33/028

(54) **Magnetfelddetektor**
Magnetic-field sensor
Détecteur de champ magnétique

(43) Veröffentlichungstag der Anmeldung: 03.03.1993
(73) Patentinhaber: Pacesetter AB, 171 95 Solna (SE)
(72) Erfinder: Högnelid, Kurt, S-137 38 Västerhanninge (SE)
(74) Vertreter: Harrison, Michael Charles

(56) Entgegenhaltungen:
- EP-A- 0 191 404
- EP-A- 0 312 605
- EP-A- 0 431 437
- GB-A- 2 070 937
- US-A- 4 186 324
- US-A- 4 887 032
- SENSOR AND ACTUATORS. vol. 16, no. 3, 1989, LAUSANNE CH pages 197 - 207; R. E. HETRICK: 'A vibrating cantilever magnetic-field sensor'

## Beschreibung

Die Erfindung betrifft einen Magnetfelddetektor für ein in dem Körper eines Patienten implantierbares medizinisches Gerät bestehend aus einem bewegungsempfindlichen Sensor, an dem eine Auswerteeinrichtung für das Signal des Sensors angeschlossen ist, einer elektrischen Spule, die an dem Sensor befestigt ist, und einer Stromquelle, die an der Spule angeschlossen ist und diese zur Detektion eines Magnetfeldes durch den Sensor und die nachgeordnete Auswerteeinrichtung mit einem elektrischen Strom beaufschlagt.

Ein aus der DE-A-2 217 400 bekannter Magnetfelddetektor besteht aus einem Magnetschalter oder Reed-Relais, der in einem Herzschrittmacher angeordnet ist. Durch Anlegen eines Magnets auf die Haut des Patienten, in dem der Herzschrittmacher implantiert ist, wird der Magnetschalter geschlossen, wodurch in dem Herzschrittmacher ein Schaltkreis zur Bestimmung der Reizschwelle des Herzens aktiviert wird.

Solange mit Hilfe des äußeren Magneten nur Testfunktionen des implantierten Gerätes wie z.B. Reizschwellenbestimmung oder Batterietest ausgelöst werden sollen, haben sich Magnetschalter in Form von Reed-Relais als ausreichend zuverlässig erwiesen. Es bestehen jedoch weitaus höhere Anforderungen an die Zuverlässigkeit und Ansprechgenauigkeit des Magnetfelddetektors, wenn durch diesen ein Behandlungsprozeß beispielsweise ein Reizimpuls, Defibrillierungschock oder die Abgabe einer Medikamentendosis durch das implantierte Gerät ausgelöst werden soll oder in einem anderen Fall inhibiert werden soll, wenn ein solcher Behandlungsprozeß aufgrund von gemessenen Parametern von dem Gerät selbsttätig ingang gesetzt wird.

Aus der US-A-4 887 032 ist ein Magnetfelddetektor bekannt, bei dem auf einem bewegungsempfindlichen piezoelektrischen Sensor eine elekrische Spule befestigt ist, die durch eine Stromquelle mit einem Wechselstrom beaufschlagt wird. Die Frequenz des Wechselstroms ist an die Resonanzfrequenz des Sensors angepaßt, so daß beim Vorhandensein eines Magnetfelds der Sensor in eine Resonanzschwingung versetzt wird wobei das Ausgangssignal des Sensors in einer Auswerteeinrichtung zur Detektion des Magnetfeldes herangezogen wird.

Der Erfindung liegt die Aufgabe zugrunde, diesen bekannten Magnetfelddetektor für seine Anwendung in einem in dem Körper eines Patienten implantierbaren medizinischen Gerät zu optimieren.

Gemäß der Erfindung wird die Aufgabe dadurch gelöst, daß bei dem Magnetfelddetektor der eingangs angegebenen Art die Stromquelle an einer Steuereinrichtung angeschlossen ist, die ein Steuersignal zum Ein- und Ausschalten der Stromquelle zu vorgegebenen Zeitpunkten erzeugt, und daß das Steuersignal der Auswerteeinrichtung zugeführt ist, die Mittel zum Verknüpfen des Signals des Sensors mit dem Steuersignal in der Weise aufweist, daß das Steuersignal das Signal des Sensors beim Einschalten der Stromquelle als Detektionssignal für ein Magnetfeld und beim Ausschalten der Stromquelle als ein der körperlichen Aktivität des Patienten entsprechendes Aktivitätssignal definiert.

Ein bewegungsempfindlicher Aktivitätssensor mit einer nachgeordneten Auswerteeinrichtung zur Aktivitätssteuerung eines Herzschrittmachers ist zwar für sich genommen beispielsweise aus der US-A-4 428 378 bekannt, jedoch sind bei dem Gegenstand der Erfindung erstmals ein Magnetfelddetektor und ein Aktivitätssensor in einem einzigen Bauelement vereinigt, so daß der in dem implantierbaren medizinischen Gerät zur Realisierung dieser beiden unterschiedlichen Sensorfunktionen benötigte Platzbedarf minimal ist. Wenn die Stromquelle zur Strombeaufschlagung der Spule ausgeschaltet ist, wird das Signal des Sensors durch das Steuersignal der Steuereinrichtung als der körperlichen Aktivität des Patienten entsprechendes Aktivitätssignal definiert. Bei eingeschalteter Stromquelle wird dagegen das Sensorsignal als Detektionssignal für ein Magnetfeld indiziert.

Vorzugsweise besteht bei dem erfindungsgemäßen Magnetfelddetektor der bewegungsempfindliche Sensor aus einem Piezokristall, der sich durch eine kleine Baugröße und ein hohes Ausgangssignal auszeichnet.

Um den von dem erfindungsgemäßen Magnetfelddetektor beim Vorliegen eines äußeren Magnetfeldes und eingeschalteter Strombeaufschlagung der Spule erzeugten magnetfeldabhängigen Signalanteil des Sensorsignals von den aktivitätsabhängigen Signalanteilen sicher unterscheiden zu können, ist es beispielsweise möglich, für den Strom durch die Spule einen bestimmten Verlauf vorzusehen, der sich in dem Signal des Sensors widerspiegelt. Hierbei hat sich ein einzelner elektrischer Stromimpuls als vorteilhaft erwiesen, weil sich das dabei in Gegenwart eines äußeren Magnetfeldes erzeugte Sensorsignal in charakteristischer Weise von den durch mechanische Stoßbeanspruchungen erzeugten Signalen unterscheidet. Dabei definiert das Steuersignal der Steueranordnung während eines vorgegebenen, über das Ende des Stromimpulses hinausgehenden Zeitintervalls das Signal des Sensors als Detektionssignal für ein Magnetfeld, so daß Nachschwingungen des Sensors in unmittelbaren Anschluß an den Stromimpuls nicht als Aktivität des Patienten mißinterpretiert werden. Schließlich ist der Stromverbrauch bei einem einzelnen Stromimpuls besonders gering.

In diesem Zusammenhang enthält die Auswerteeinrichtung des erfindungsgemäßen Magnetfelddetektors vorteilhafterweise einen Frequenzdiskriminator und einen Schwellenwertdetektor zur Überwachung des Sensorsignals auf Überschreiten einer vorgegebenen Signalschwelle bei einer der Eigenfrequenz des Sensors entsprechenden Frequenz. Aufgrund des einzelnen Stromimpulses wird nämlich der bewegungsempfindliche Sensor beim Vorliegen eines äußeren Magnetfeldes durch einen einzelnen kurzen Kraftstoß mechanisch angeregt, um dann mit seiner Eigenfrequenz unbehindert auszuschwingen. Das dementsprechende Sensorsignal ist eine abklingende Schwingung mit der Eigenfrequenz des Sensors und unterscheidet sich somit deutlich von sonstigen Störsignalen.

Die Sicherheit bei der Magnetfelddetektion läßt sich in vorteilhafter Weise noch dadurch erhöhen, daß der Frequenzdiskriminator bei der Beaufschlagung der Spule mit dem elektrischen Strom während des vorgegebenen Zeitintervalls aktiviert wird, so daß die Detektion eines Magnetfeldes zeitlich auf die Dauer des Zeitintervalles beschränkt ist.

Zur Auswertung des Aktivitätsignals ist vorzugsweise an dem Sensor eine Signalverarbeitungseinrichtung für das Aktivitätssignal angeschlossen, die bei der Beaufschlagung der Spule mit dem elektrischen Strom während der vorgegebenen Zeitdauer inhibiert wird.

Zur Erläuterung der Erfindung wird im folgenden auf die Figuren der Zeichung Bezug genommen; im einzelnen zeigen
- Fig 1: ein Ausführungsbeispiel für den bewegungsempfindlichen Sensor mit einer daran befestigen Spule,
- Fig 2: eine Blockschaltbild des erfindungsgemäßen Magnetfelddetektors und
- Fig 3: Beispiele für den Strom durch die Spule und die daraus resultierende Sensorspannung bei fehlendem und bei vorhandenem äußeren Magnetfeld.

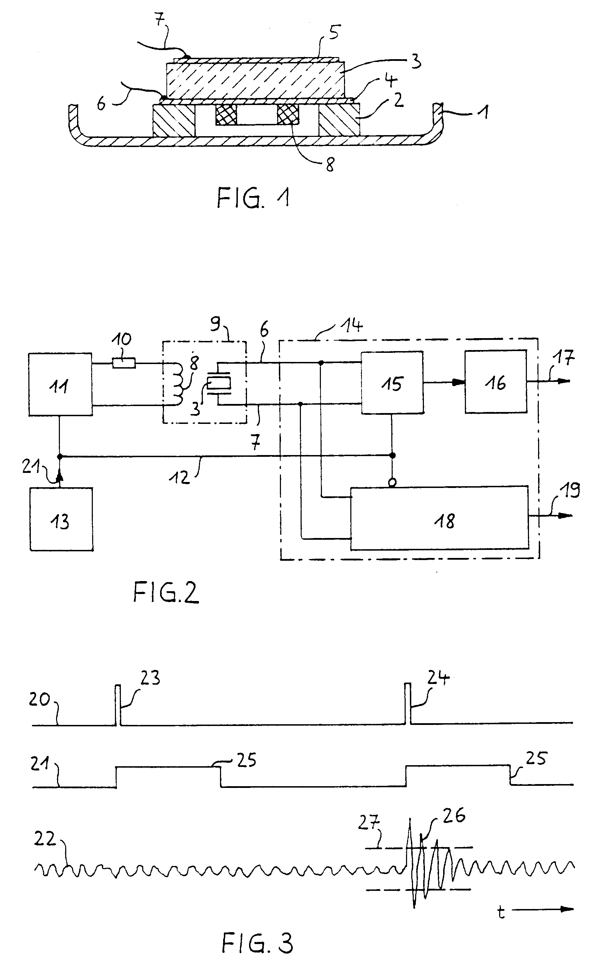

Fig 1 zeigt in schematischer Darstellung einen Schnitt durch einen Teil des Gehäuses 1 eines implantierbaren Defibrillators oder sonstigen implantierbaren medizinischen Geräts. Auf der Innenwand des Gehäuses 1 ist ein Distanzring 2 aufgeklebt, auf dem ein scheibenförmiger piezoelektrischer Sensor 3 aufgeklebt ist, der beidseitig mit Kontaktelektroden 4 und 5 versehen ist. Die beiden Kontaktelektroden 4 und 5 sind mit Anschlüssen 6 bzw. 7 verbunden, zwischen denen die in dem piezoelektrischen Sensor 3 erzeugte elektrische Spannung abgreifbar ist. Für den Fall, daß der Distanzring 2 und das Gehäuse 1 jeweils aus einem elektrisch leitenden Material bestehen, kann die Spannung auch zwischen der mit 5 bezeichneten Kontaktelektrode und dem Gehäuse 1 abgegriffen werden. In dem Hohlraum, der von der Kontaktelektrode 4, dem Gehäuse 1 und der Innenseite des Distanzringes 2 gebildet wird, ist eine elektische Spule 8 angeordnet, die durch Aufkleben auf die Kontaktelektrode 4 mit dem piezoelektrischen Sensor 3 fest verbunden ist. Die elektrische Spule 8 kann auch auf gleiche Weise auf der gegenüberliegenden Seite des piezoelekrischen Sensors 3 mit diesem verbunden sein. Bei dem dargestellten Ausführungsbeispiel ist die elektrische Spule 8 als Drahtspule ausgeführt; es ist aber genauso möglich, eine oder beide der Kontaktelektroden 4 und 5 in Form von spiralförmigen Leiterbahnen auszubilden, die auf diese Weise die elektrische Spule bilden.

In der Fig 2 ist in einem vereinfachten Blockschaltbild die schaltungstechnische Anordnung des piezoelektrischen Sensors 3 und der Spule 8 zu einem Magnetfelddetektor dargestellt. Dabei ist durch die strichpunktierte Umrandung 9 angedeutet, daß die elektrische Spule 8 und der piezoelektrische Sensor 3 fest miteinander verbunden sind. Die Spule 8 ist über einen Widerstand 10 an einer steuerbaren Stromquelle 11 angeschlossen. Die Stromquelle 11 ist über eine Steuerleitung 12 mit einer Steuereinrichtung 13 verbunden, die in vorgegebenen, vorzugsweise programmierbaren Zeitabständen die Stromquelle 11 zur Abgabe eines rechteckförmigen Stromimpulses aktiviert. An dem Sensor 3 ist eine Auswerteeinrichtung 14 angeschlossen, innerhalb derer ein Frequenzdiskriminator 15, der auf die Eigenfrequenz des piezoelektrischen Sensors 3 eingestellt ist, eingangsseitig mit den Anschlüssen 6 und 7 des Sensors 3 verbunden ist. Der Frequenzdiskriminator 15 wird über die Steuerleitung 12 gleichzeitig mit der Aktivierung der Stromquelle 11 für die Dauer eines vorgegebenen Zeitintervalles aktiviert. Dem Frequenzdiskriminator 15 ist ein Schwellenwertdetektor 16 nachgeordnet, der einen Ausgang 17 aufweist. Wie untenstehend noch an Hand von Fig 3 näher erläutert wird, wird beim Vorhandensein eines äußeren Magnetfeldes als Folge des von der Stromquelle 11 abgegebenen Stromimpulses in dem piezoelektrischen Sensor 3 ein Sensorsignal erzeugt, das die Auswerteeinrichtung 14 zur Abgabe eines Ausgangssignales an ihrem Ausgang 17 veranlasst.

An den Anschlüssen 6 und 7 des piezoelektrischen Sensors 3 ist ferner eine Signalverarbeitungseinrichtung 18 zur Erfassung von Sensorsignalen angeschlossen, die der körperlichen Aktivität eines das implantierbare Gerät tragenden Patienten entsprechen. Die Signalverarbeitungseinrichtung 18 weist einen Ausgang 19 auf und wird von der Steuereinrichtung 13 über die Steuerleitung 12 für die Dauer des vorgegebenen Zeitintervalles inhibiert, währenddessen die Auswerteeinrichtung 14 aktiviert ist.

In Fig 3 ist mit 20 der Strom durch die Spule 8, mit 21 das von der Steuereinrichtung 13 über die Steuerleitung 12 abgegebene Steuersignal und mit 22 das Sensorsignal des piezoelekrischen Sensors 3 bezeichnet. Wie der mit 20 bezeichnete Kurvenverlauf zeigt, wird die Stromquelle 11 von der Steuereinrichtung 13 in regelmäßigen Zeitabständen von beispielsweise einer Sekunde zur Abgabe eines Stromimpulses 23, 24 angesteuert. Gleichzeitig mit der Erzeugung der Stromimpulse 23, 24 wird der Frequenzdiskriminator 15 durch das Steuersignal 21 für die Dauer eines Zeitintervalles 25 aktiviert und gleichzeitig die Signalverarbeitungseinrichtung 18 für dieselbe Dauer inhibiert. Wenn wie im Falle des mit 23 bezeichneten Stromimpulses kein äußeres Magnetfeld vorliegt, bleibt der piezoektrische Sensor 3 von dem Stromimpuls 23 unbeeinflußt und erzeugt an seinen beiden Anschlüssen 6 und 7 ein der momentanen körperlichen Aktivität des Patienten entsprechendes Sensorsignal. Dieses Sensorsignal 22 wird außerhalb der Zeitintervalle 25 durch die Signalverarbeitungseinrichtung 18 in einer Weise verarbeitet, wie sie beispielsweise aus der oben bereits erwähnten US-A-4 428 378 bekannt ist.

Im Falle des mit 24 bezeichneten Stromimpulses sei angenommen, daß ein äußeres Magnetfeld vorliegt, so daß durch das äußere Magnetfeld und das durch den Strom in der Spule 8 erzeugte Magnetfeld eine Kraft auf die Spule 8 ausgeübt wird, die auf den mit ihr verbundenen piezoelektrischen Sensor 3 übertragen wird und in diesem ein charakteristisches Sensorsignal 26 erzeugt. Im Falle des rechteckförmigen Stromimpulses 24 wirkt auf den piezoelektrischen Sensor 3 ein Kraftstoß, der den Sensor 3 zu einer gedämpften Schwingung mit der Eigenfrequenz des Sensors 3 anregt. In dem Frequenzdiskriminator 15 wird das Sensorsignal 26 auf diese vorbestimmte Eigenfrequenz hin überwacht und von dem Schwellenwertdetektor 16 ein Ausgangssignal erzeugt, wenn die Schwingung mit der Eigenfrequenz einen vorgegebenen Schwellenwert 27 überschreitet.

## Patentansprüche

1. Magnetfelddetektor für ein in dem Körper eines Patienten implantierbares medizinische Gerät bestehend aus einem bewegungsempfindlichen Sensor (3), an dem eine Auswerteeinrichtung (14) für das Signal (26) des Sensors (3) angeschlossen ist, einer elektrischen Spule (8), die an dem Sensor (3) befestigt ist, und einer Stromquelle (11), die an der Spule (8) angeschlossen ist und diese zur Detektion eines Magnetfeldes durch den Sensor (3) und die nachgeordnete Auswerteeinrichtung (14) mit einem elektrischen Strom (23,24) beaufschlagt, **dadurch gekennzeichnet**, daß die Stromquelle (11) an einer Steuereinrichtung (13) angeschlossen ist, die ein Steuersignal (21) zum Ein- und Ausschalten der Stromquelle (11) zu vorgegebenen Zeitpunkten erzeugt, und daß das Steuersignal (21) der Auswerteeinrichtung (14) zugeführt ist, die Mittel zum Verknüpfen des Signals (22,26) des Sensors (3) mit dem Steuersignal in der Weise aufweist, daß das Steuersignal (21) das Signal (22,26) des Sensors (3) beim Einschalten der Stromquelle (11) als Detektionssignal (26) für ein Magnetfeld und beim Ausschalten der Stromquelle (11) als ein der körperlichen Aktivität des Patienten entsprechendes Aktivitätssignal (22) definiert.

2. Magnetfelddetektor nach Anspruch 1, **dadurch gekennzeichnet**, daß der bewegungsempfindliche Sensor (3) ein piezoelektrischer Sensor ist.

3. Magnetfelddetektor nach Anspruch 1 oder 2, **dadurch gekennzeichnet**, daß die Stromquelle (11) beim Einschalten durch die Steuereinrichtung (13) einen elektrischen Stromimpuls (23,24) erzeugt und daß das Steuersignal (21) der Steuereinrichtung (13) während eines vorgegebenen, über das Ende des Stromimpulses (23,24) hinausgehenden Zeitintervalls (25) das Signal (22,26) des Sensors (3) als Detektionssignal (26) für ein Magnetfeld definiert.

4. Magnetfelddetektor nach Anspruch 3, **dadurch gekennzeichnet**, daß die Auswerteeinrichtung (14) einen Frequenzdiskriminator (15) und einen Schwellenwertdetektor (16) zur Überwachung des Sensorsignals (22,26) auf Überschreiten einer vorgegebenen Signalschwelle (27) bei einer der Eigenfrequenz des Sensors (3) entsprechenden Frequenz enthält.

5. Magnetfelddetektor nach Anspruch 4, **dadurch gekennzeichnet**, daß der Frequenzdiskriminator (15) bei der Beaufschlagung der Spule (8) mit dem elektrischen Strom (23,24) während des vorgegebenen Zeitintervalls (25) aktiviert wird.

6. Magnetfelddetektor nach einem der Ansprüche 3 bis 5, **dadurch gekennzeichnet**, daß an dem Sensor (3) eine Signalverarbeitungseinrichtung (18) für das Aktivitätssignal (22) angeschlossen ist, die bei der Beaufschlagung der Spule (8) mit dem elektrischen Strom (23,24) während der vorgegebenen Zeitdauer (25) inhibiert wird.

## Claims

1. Magnetic field detector for a medical apparatus which can be implanted in the body of a patient, comprising a movement sensitive sensor (3), to which is connected an evaluating device (14) for the signal (26) of the sensor (3), an electric coil (8), which is secured to the sensor (3), and a current source (11), which is connected to the coil (8) and applies to the latter an electric current (23, 24) for the detection of a magnetic field by the sensor (3) and the subsequently arranged evaluating device (14), characterised in that the current source (11) is connected to a control device (13), which produces a control signal (21) for switching the current source (11) on and off at pre-determined time instants, and in that the control signal (21) is supplied to the evaluating device (14), which has means for combining the signal (22, 26) of the sensor (3) with the control signal in such a way that when the current source (11) is switched on, the control signal (21) defines the signal (22, 26) of the sensor (3) as a detection signal (26) for a magnetic field, and when the current source (11) is switched off, the control signal (21) defines the signal (22, 26) of the sensor (3) as an activity signal (22) corresponding to the physical activity of the patient.

2. Magnetic field detector according to claim 1, characterised in that the movement sensitive sensor (3) is a piezoelectric sensor.

3. Magnetic field detector according to claim 1 or 2, characterised in that when the current source (11) is switched on by means of the control device (13), it produces an electric current pulse (23, 24), and in that during a pre-determined time interval (25) which extends beyond the end of the current pulse (23, 24), the control signal (21) of the control device (13) defines the signal (22, 26) of the sensor (3) as a detection signal (26) for a magnetic field.

4. Magnetic field detector according to claim 3, characterised in that the evaluating device (14) contains a frequency discriminator (15) and a threshold value detector (16) for monitoring the sensor signal (22, 26) for the exceeding of a pre-determined signal threshold (27) in the case of a frequency which corresponds to the natural frequency of the sensor (3).

5. Magnetic field detector according to claim 4, characterised in that when the electric current (23, 24) is applied to the coil (8), the frequency discriminator (15) is activated during the pre-determined time interval (25).

6. Magnetic field detector according to one of the claims 3 to 5, characterised in that connected to the sensor (3) there is a signal processing device (18) for the activity signal (22), which signal processing device, when the electric current (23, 24) is applied to the coil (8), is inhibited during the pre-determined time span (25).

## Revendications

1. Détecteur de champ magnétique pour un appareil médical implanté dans le corps d'un patient, consistant en un capteur (3) sensible au déplacement, auquel est raccordée une installation (14) d'exploitation du signal (26) du capteur (3), en une bobine (8) électrique, qui est fixée au capteur (3) et en une source (11) de courant, qui est raccordée à la bobine (8) et qui l'alimente en un courant électrique (23, 24) en vue de la détection d'un champ magnétique par le capteur (3) et par l'installation (14) d'exploitation qui est montée en aval, caractérisé en ce que la source de courant est raccordée à une installation (13) de commande qui produit, à des instants donnés à l'avance, un signal (21) de commande en vue de connecter et de déconnecter la source (11) de courant, et en ce que le signal (21) de commande est envoyé à l'installation (14) d'exploitation qui comporte des moyens destinés à combiner le signal (22, 26) du capteur (3) au signal de commande, de manière que le signal (21) de commande définisse le signal (22, 26) du capteur (3) comme signal (26) de détection d'un champ magnétique, lorsque la source (11) de courant est connectée, et comme un signal (22) d'activité correspondant à l'activité corporelle du patient, lorsque la source (11) de courant est déconnectée.

2. Détecteur de champ magnétique suivant la revendication 1, caractérisé en ce que le capteur (3) sensible au déplacement est un capteur piézoélectrique.

3. Détecteur de champ magnétique suivant la revendication 1 ou 2, caractérisé en ce que la source (11) de courant produit, lorsqu'elle est connectée par l'installation (13) de commande, une impulsion (23, 24) électrique de courant et en ce que le signal (21) de commande de l'installation (13) de commande définit, pendant un intervalle (25) de temps donné à l'avance et commençant à la fin de l'impulsion (23, 24), le signal (22, 26) du capteur (3) comme signal (26) de détection d'un champ magnétique.

4. Détecteur de champ magnétique suivant la revendication 3, caractérisé en ce que l'installation (14) d'exploitation comporte un discriminateur (15) de fréquence et un détecteur (16) de seuil servant à surveiller que le signal (22, 26) du capteur ne dépasse pas un seuil (27) de signal donné à l'avance pour une fréquence correspondant à la fréquence propre du capteur (3).

5. Détecteur de champ magnétique suivant la revendication 4, caractérisé en ce que le discriminateur (15) de fréquence est activé, lors de l'alimentation de la bobine (8) par le courant (23, 24) électrique, pendant l'intervalle (25) de temps donné à l'avance.

6. Détecteur de champ magnétique suivant l'une des revendications 3 à 5, caractérisé en ce qu'une installation (18) de traitement du signal (22) d'activité est raccordée au capteur (3), cette installation étant inhibée lors de l'alimentation de la bobine (8) par le courant (23, 24) électrique, pendant la durée (25) donnée à l'avance.
